# EUROPEAN PATENT SPECIFICATION

(11) **EP 2 129 298 B1**
(45) Date of publication and mention of the grant of the patent: **09.09.2015**
(21) Application number: 08701947.7
(22) Date of filing: 25.01.2008
(51) Int. Cl.: A61B 17/00, A61M 5/31

(54) **HAEMOSTAT APPLICATION**
HÄMOSTAT-ANWENDUNG
POSE D'UN DISPOSITIF HÉMOSTATIQUE

(30) Priority: 26.01.2007 GB 0701496
(43) Date of publication of application: 09.12.2009
(73) Proprietor: Medtrade Products Limited, Cheshire CW1 6GL (GB)
(72) Inventor: HARDY, Craig, Julian, Cheshire CW3 0AY (GB); EASON, Guy, Cheshire CW2 7NE (GB); SHARAFANOWICH, Kathryn, Island, WA 98110 (US)
(74) Representative: Wilson Gunn
(86) International application number: PCT/GB2008/000274
(87) International publication number: WO 2008/090364

(56) References cited:
- EP-A- 0 252 214
- WO-A-00/71032
- US-A- 5 192 300
- US-A1- 2003 018 357
- US-B1- 6 200 328

## Description

This invention relates to the field of application of haemostats to sites of bleeding, in order to control, reduce or end bleeding, particularly internal blood loss.

In particular, the invention relates to a device for the insertion of a haemostat to a deep site of bleeding.

Haemostats are used in medical treatment to staunch bleeding wounds due to injury, or surgical incisions. They act by physical or chemical means to reduce or eliminate blood loss e.g. by absorption of liquid, or by promoting clotting or coagulation or both. The haemostat itself may be in the form of a powder, or fibrous material for example, including dry foam, freeze dried foam, or a fibrous nonwoven, a gel or the like. An absorbent material may be used as a support for a chemically active material distributed through the absorbent material.

At present, such haemostat preparations are applied using dressings for surface tissue wounds, or by specially adapted forceps or tongs, the latter having relatively long limbs to assist in placing haemostats in deep tissues.

Such existing methods are effective with wounds which are sufficiently wide to allow manipulation with such tools but small aperture deep wounds, such as may be produced by gunshot or stabbing cause difficulty, and may require to be surgically widened to allow placement of the haemostat.

US 5,192,300 discloses a tool for applying a haemostat comprising the features defined in the preamble of claim 1. However, this tool does not permit easy loading of the haemostat.

It is an object of the invention to provide a tool which can be used to locate haemostats in their correct place in deep or narrow or other wounds or incisions which are difficult of access.

In accordance with the invention, a tool for application of a haemostat comprises a barrel for holding a quantity of haemostat, an outlet, and a plunger operable to expel the haemostat from the barrel, wherein the barrel has an inner sleeve and an outer sleeve, a loading aperture in each of the sleeves, the sleeves being relatively rotatable whereby the apertures may alternatively be placed in alignment or out of alignment, one with the other; a plunger which can be moved axially of the barrel between a withdrawn position and a fully inserted position, and an outlet at an end of the barrel opposite to the plunger, said outlet being of at least minimally smaller diameter than the internal diameter of the barrel, to provide an abutment to limit full insertion of the plunger.

The loading apertures when aligned preferably admit to a chamber within the barrel, for placement of a haemostat therein when the plunger is fully withdrawn. The haemostat preparation may be provided in the form of a cartridge or pellet of a size and configuration to fit into the chamber, and may be provided in a bag or casing which is preferably of soluble material.

The haemostat may alternatively be in the form of a cylindrical plug or fibrous material. This can contain granules of any chemically or pharmaceutically active composition, in the quantities prescribed, which may form a minor part of the haemostat by weight. The material of the fibrous plug may be inactive, or have at least physical haemostatic action, e.g. as an absorbent.

The outlet may be defined by an internal rim about the respective end of the barrel which is sufficient to provide a step to retain the plunger against being pushed beyond the respective end of the barrel.

The loading apertures may comprise elongate slots one in each of the inner and outer sleeves. The inner sleeve may be fixed with respect to an end member which has an aperture for sliding of the plunger, and the shank of the plunger may be of any suitable shape such as cruciform, or cylindrical. The outlet may alternatively be formed as for example a rounded, tapering or conical nozzle, with an opening of reduced diameter with respect to the diameter of the barrel and optionally with longitudinal slots in a cruciform array. This may be of particular utility for the placement of a particulate, powdered, or pelletized haemostat preparation, whilst a wider outlet aperture such as suggested above, may be of particular utility for location of haemostatic preparations provided in the form of bagged doses, or as fibrous plugs or wads. Such nozzles may be interchangeable to provide orifices of differing size and shape, may be fittable over the full width end of the barrel. The longitudinal slots in the outlet end or cone may extend back beyond any tapered section of the end so that the end can open out in the manner of the petals of a flower.

To penetrate into wounds having small or very small entry sizes, e.g. stab wounds or gunshot pellet wounds, the barrel of the tool preferably has a diameter of 2.0cm or less, preferably 1.5cm or less, more preferably 1.0cm or less, most preferably 0.5cm or less.

Preferred embodiments of tool for haemostat application according to the invention will now be described, by way of example, with reference to the accompanying drawings, wherein:-
- Fig. 1: is a perspective view of a first embodiment of applicator tool according to the invention;
- Fig. 2: is a longitudinal cross-sectional view of the applicator tool of Fig. 1;
- Fig. 3: is a longitudinal cross-sectional view similar to Fig. 2 of a modified version of the applicator tool;
- Fig. 4: is a fragmentary perspective view of an alternative nozzle outlet;
- Fig. 5: is a perspective view of a further embodiment of applicator tool according to the invention;
- Fig. 6: is a longitudinal cross-sectional view of the applicator tool of Fig. 5; and
- Fig. 7: is a longitudinal cross-sectional view of a simplified applicator tool according to the invention.

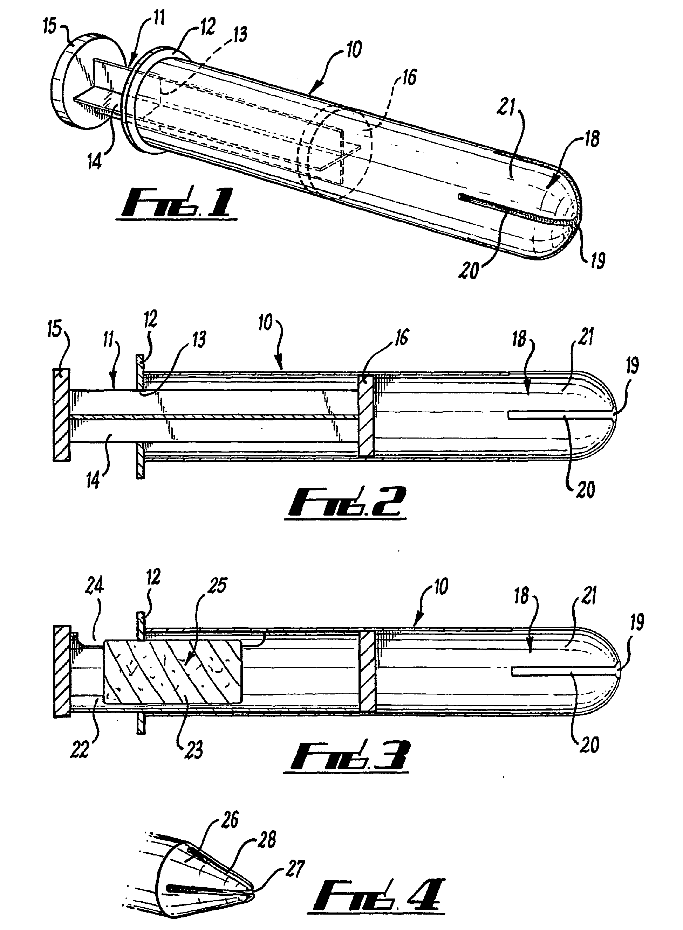

The applicator tool shown in Figs. 1 and 2 comprises a cylindrical barrel 10, and a plunger 11 which is inserted slidably into the barrel 10 from a first end of the barrel. The first end of the barrel is closed by a disc 12 which has a guide aperture 13, of a cruciform shape to match a cruciform cross-sectional shaft 14 of the plunger 11. The free end of the plunger 11 is formed with a disc or knob 15, whilst the end of the plunger 11 located in the barrel 10 forms a disc shaped piston 16, dimensioned to slidingly contact the inner surface of the barrel 10. This piston 16 prevents withdrawal of the plunger 11 from the barrel 10 when fully extended, and serves to propel a dose of e.g. haemostatic material from the discharge end of the barrel 10.

The discharge end of the barrel 10 is constituted by the end 17 of the barrel opposite the first end of the barrel which is closed by disc 12. The discharge end 17 is formed as a torpedo shaped end 18 with an end orifice 19, and longitudinal slots 20 which divide the rounded end cap into tapering segments 21. The material of these segments is a flexible plastic, so that the segments may dilate like the petals of a flower to expand the orifice 19 if relatively large bodies such as 25 in Fig. 3 of material are expelled.

A quantity of material may be loaded into the barrel 10 in the manner of a syringe, by withdrawing the plunger 11 and allowing the material to be drawn in by suction. This is suitable for use with powders, liquids, or fluent gels. To load more solid or fibrous material say in the form of pellets or wads, the flexible segments 21 may be opened out, and the material pushed into the barrel by the user's gloved fingers, or by a suitable tool such as a spatula.

The tool is operated to discharge the material at the required site simply be pressing the plunger into the barrel, so that the piston 16 will push the material out through the orifice 19.

The Fig. 3 embodiment differs from that of Figs. 1 and 2, in that the plunger 11 is configured as a cylinder 22, enclosing a chamber 23 which is accessible through an opening 24 in the wall of the cylinder. The chamber is used to store one or more charges of haemostat material 25, which may be ready made up into bags sachets wads or plugs of material. The bags may be of water or body fluid soluable or dispersable material, and contain the haemostatic material, whilst wads or plugs may be of compacted granular material, or of fibrous material which is either inert or has a haemostatic effect itself, and may carry medically active material dispensed therethrough, as granular material or as an impregnating fluid.

The bags, sachets wads or plugs 25 may be loaded into the barrel 10 in the same way as in the first embodiment, as the discharge end 17 is configured in the same way as in Figs. 1 and 2, and corresponding parts have the same reference numerals as in the foregoing.

Fig. 4 shows an alternative configuration for the discharge end of the tool, which comprises a conical end cap 26, which has a similar end orifice 27 and similar longitudinal slots 28.

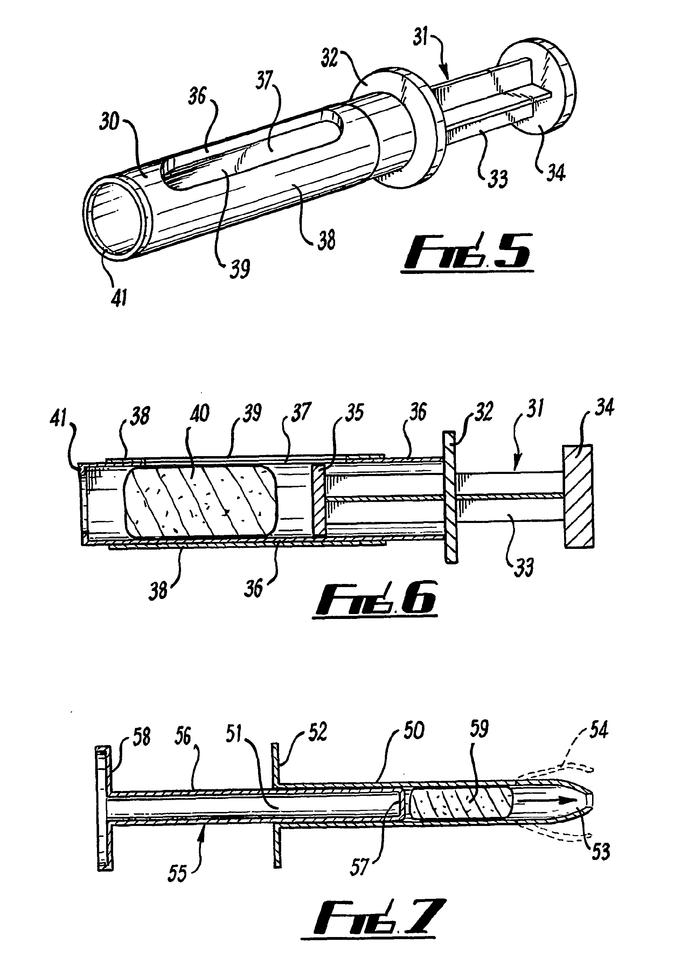

A further embodiment of tool for application of haemostats or other medical or pharmaceutical devices or doses to a site, is shown in Figs. 5 and 6. The tool comprises a barrel 30 and a plunger 31. The barrel is configured as a cylinder, and has a first end which is closed by a disc 32 having a cruciform aperture for sliding engagement of a cruciform shank 33 of the plunger 32. The free end of the plunger 32 is formed with an end disc or knob 34, and the inner end, contained within the barrel 30, carries a circular disc 35 which acts as a piston.

The barrel 30 comprises an inner, fixed cylindrical wall 36, having a slot 37 extending therealong. The barrel 30 is further provided with an outer cylindrical sleeve 38 which can be rotated by twisting the sleeve relative to the inner wall 36. The sleeve 38 has a slot 39 extending therealong which is congruent with the slot 37 in the wall 36 and can be aligned with the latter or closed relative thereto, by rotation of the sleeve. The sleeve 38 can be rotated by simple hand pressure, but an annular part thereof may be textured or provided with ribs or knurling to provide a finger grip.

The slots 37, 39 may be aligned to provide an opening through which a charge 40 of haemostat material may be inserted into the barrel 30. This may be provided in a soluble bag, as a wad or plug of fibrous, or gel material etc. The opening is closed simply by rotating the sleeve 38 until the aperture slots are no longer aligned.

The end 41 of the barrel 30 opposite to the plunger 31 provides a discharge opening 42 which is surrounded by a rim 43 which provides an abutment to prevent excess travel of the piston 35, so that the opening 42 is only slightly smaller in diameter than the internal diameter of the barrel 30. This is particularly suitable for discharge of bags or plugs of material of diameter comparable to that of the barrel.

The outlet configuration may be varied by fitting appropriate cones over the end of the barrel, and the discharge opening structure described in relation to this embodiment may be the basic structure, for the Fig. 1 - 2 or Fig. 3 embodiment also, and be adaptable for discharge of powders, particles or gels for example by fitting appropriate nozzle cones such as described in relation to the foregoing embodiments. These cones may vary as to outlet size and shape, for example for extruding a plastic gel or paste into the required site.

Figure 7 illustrates a simplified haemostat applicator which is capable of being used as a disposable one-slot applicator or very simple mass producible item. The applicator is formed from a plastics material, and comprises a tubular barrel 50 having an open end 51 with a flange 52, and a discharge end 53 with a dilatable slotted end cone 54. The applicator also comprises a hollow plastics plunger, comprising a shaft 55 comprising a hollow tube 56 with a closed inner end 57, and an end flange 58 which is used to aid pressure. In use a charge 59 of haemostatic material is inserted into the open end 51 of the barrel 50, and pushed down towards the discharge end 53 using the plunger 55, which is inserted into the open end 51 after the charge 59. To carry out treatment, the barrel 50 is then inserted into the wound to penetrate to the site of bleeding, and the charge 59 of haemostatic material is implanted by pushing the plunger 55 completely home, till flange 58 abuts flange 52.

The pressure expels the charge 59 from the discharge end 53 causing the segments of the cone 54 to dilate into the broken line positions 54a, to allow the charge to be expelled from the end of the barrel 50.

The tools described may be made suitable for disassembly for sterilization and re-use or may be disposable after one use.

The barrel, plunger and any other parts may be made from suitable inert plastics materials, and may be provided, ready charged with a specific haemostat material or combination materials, sealed to maintain sterility until use. The material is preferably transparent.

As the tool is of relatively small diameter, it can be inserted into wounds or incisions of small diameter, and implant the haemostat into relatively deep bleeding sites without need for the wound to be widened surgically.

The haemostat may be adapted to expand after placement at the required site, and release from the confines of the tube. The degree of expansion, by volume, or by linear measure, may be greater than 10%, preferably more than 33%, and ideally between 50 and 100%, although excessive expansion might damage body tissues.

A preferred material for the barrel and plunger is a polyolefine such as polypropylene, or high, medium, or low-density polyethylene, or a polyester. Advantageously a biodegradable plastic may be used, so that if the tool is discarded in the field for instance, it will biodegrade.

The tool could be formed with a rounded tip to allow easier access to a small site such as a bullet entery wound.

The tool may be used as part of an automated system to be dispensed at the touch of a button, for example using air under pressure.

The tool, particularly the outer surface of the barrel, may have a lubricated self-lubricating, or low friction surface or coating to ease insertion and reduce pain produced by abrasion during insertion.

The barrel may be calibrated so that the user can judge the amount of material applied and thus judge the size of an internal cavity which cannot be seen.

The tool is suitable to be carried by paramedics, and by military casualty treatment teams, for use in applying immediate treatment to injuries, and combat wounds, and may also be used in casualty wards and surgically for treatment e.g. of bleeding in 'keyhole' incisions.

The barrel of the syringe may be made of a material which is dissoluble in body fluids such as blood plasma etc and is left in place without being withdrawn, and may be dissolved in situ. This may make the applicator more effective, easier or faster to use, or be necessary if a soluble bag or some of a haemostatic powder adheres to the rim of the barrel when the latter is wetted.

Where the barrel is filled with loose unbagged powder, this may be retained in place with a soluble haemostatic discardable or removable plug.

The tool may be adaptable for other medical purposes such as the placement of suppositories or the like in target locations.

## Claims

1. A tool for application of a haemostat comprising a barrel (30) for holding a quantity of a haemostat, an outlet (42), and a plunger (31) operable to expel the haemostat from the barrel, the barrel has an inner sleeve (36) and an outer sleeve (38), **characterised in that** a loading aperture (37,39) is provided in each of the sleeves, the sleeves being relatively rotatable whereby the apertures may alternatively be placed in alignment or out of alignment one with the other; a plunger (31) which can be moved axially of the barrel between a withdrawn position and a fully inserted position, and an outlet (42) at one end (41) of the barrel (30) opposite to the plunger (31), said outlet being of at least minimally smaller diameter than the internal diameter of the barrel (30), to provide an abutment to limit full insertion of the plunger (31).

2. A tool according to claim 1, wherein the loading apertures when aligned admit to a chamber within the barrel (30), for placement of a haemostat therein when the plunger (31) is fully withdrawn.

3. A tool according to claim 1 or 2, wherein the outlet (42) is defined by an internal rim (43) about the respective end of the barrel (30) which provides a step to retain the plunger (31) against being pushed beyond the respective end of the barrel (30).

4. A tool according to any of claims 1 to 3, wherein the loading apertures comprise elongate slots (37, 39) one in each of the inner and outer sleeves (36, 38) the inner sleeve (36) being fixed with respect to an end member which has an aperture for sliding of the plunger (31).

5. A tool according to any of claims 1 to 4, wherein the outlet (42) is formed as a rounded, tapering or conical nozzle with an opening of reduced diameter with respect to the diameter of the barrel (30).

6. A tool according to claim 5, wherein the nozzle is formed with longitudinal slots in a cruciform array converging on the opening.

7. A tool according to any preceding claim wherein the outer surface of the barrel (30) has a coating of self lubricating or low friction material.

8. A tool according to any preceding claim wherein the barrel (30) is calibrated so that the user can judge the amount of material applied.

9. A tool according to any preceding claim wherein the barrel (30) is made of a material which is dissoluble in body fluids such as blood plasma.

10. A tool according to claim 1, wherein an open ended barrel (30) is provided with a withdrawable plunger, which can be inserted into the open end, after a charge of haemostatic material, the barrel (30) having a discharge end opposite said open end.

11. A tool according to claim 10, wherein the discharge end includes a cone with longitudinal slots which provide flaps which can be dilated to allow egress of a body of haemostatic material.

12. A tool according to any preceding claim, wherein the barrel (30) has a diameter of 2cm or less.

13. A tool according to claim 12 wherein the barrel (30) has a diameter of 1.5cm or less.

14. A tool according to claim 13 wherein the barrel (30) has a diameter of 1.0cm or less.

15. A tool according to claim 14 wherein the barrel (30) has a diameter of 0.5cm or less.

## Patentansprüche

1. Werkzeug zur Applikation eines Hämostyptikums, umfassend einen Zylinder (30) zur Aufnahme einer Menge eines Hämostyptikums, einen Auslass (42) und einen Kolben (31), der dazu ausgelegt ist, das Hämostyptikum aus dem Zylinder auszutreiben, wobei der Zylinder eine Innenhülse (36) und eine Außenhülse (38) aufweist, **dadurch gekennzeichnet, dass** in jeder der Hülsen eine Ladeöffnung (37, 39) bereitgestellt wird, wobei die Hülsen relativ zueinander drehbar sind, wobei die Öffnungen alternativ in Überdeckung oder Nicht-Überdeckung mit einander gebracht werden können, einen Kolben (31), der axial in Bezug auf den Zylinder zwischen einer zurückgezogenen Stellung und einer vollständig eingeführten Stellung bewegt werden kann, und einen Auslass (42) an einem Ende (41) des Zylinders (30) gegenüber dem Kolben (31), wobei der Auslass einen mindestens minimal kleineren Durchmesser als der Innendurchmesser des Zylinders (30) aufweist, um ein Widerlager bereitzustellen, um die vollständige Einführung des Kolben (31) zu begrenzen.

2. Werkzeug nach Anspruch 1, wobei die Ladeöffnungen im miteinander überdeckenden Zustand den Zugang zu einer Kammer innerhalb des Zylinders (30) ermöglichen, um ein Hämostyptikum in diesen hinein zu füllen, wenn der Kolben (31) vollständig zurückgezogen ist.

3. Werkzeug nach Anspruch 1 oder 2, wobei der Auslass (42) durch einen Innenrand (43) um das betreffende Ende des Zylinders (30) herum festgelegt wird, wodurch eine Stufe bereitgestellt wird, um den Kolben (31) davon abzuhalten, über das entsprechende Ende des Zylinders (30) hinaus gedrückt zu werden.

4. Werkzeug nach einem der Ansprüche 1 bis 3, wobei die Ladeöffnungen Längsschlitze (37, 39) aufweisen, jeweils einen in der Innenhülse und der Außenhülse (36, 38), wobei die Innenhülse (36) in Bezug auf ein Endbauteil, das eine Öffnung zum Verschieben des Kolbens (31) aufweist, fixiert ist.

5. Werkzeug nach einem der Ansprüche 1 bis 4, wobei der Auslass (42) in Form einer abgerundeten, sich verjüngenden oder konischen Düse mit einer Öffnung mit einem kleineren Durchmesser als der Durchmesser des Zylinders (30) ausgebildet ist.

6. Werkzeug nach Anspruch 5, wobei die Düse mit Längsschlitzen in einer kreuzförmigen Anordnung, die zu der Öffnung hin konvergieren, ausgebildet ist.

7. Werkzeug nach einem der vorhergehenden Ansprüche, wobei die Außenfläche des Zylinders (30) einen Überzug aus selbst-schmierendem oder reibungsarmem Material aufweist.

8. Werkzeug nach einem der vorhergehenden Ansprüche, wobei der Zylinder (30) kalibriert ist, sodass der Anwender die Menge des applizierten Materials beurteilen kann.

9. Werkzeug nach einem der vorhergehenden Ansprüche, wobei der Zylinder (30) aus einem Material hergestellt ist, das in Körperflüssigkeiten, wie Blutplasma, unlöslich ist.

10. Werkzeug nach Anspruch 1, wobei ein offen-endiger Zylinder (30) mit einem zurückziehbaren Kolben ausgestattet ist, der nach einer Beladung des hämostyptischen Materials in das offene Ende eingeführt werden kann, wobei der Zylinder (30) gegenüber dem offenen Ende ein Auslassende aufweist.

11. Werkzeug nach Anspruch 10, wobei das Auslassende einen Kegel mit Längsschlitzen aufweist, wodurch Laschen bereitgestellt werden, die erweitert werden können, um das Austreten einer Menge hämostyptischen Materials zu ermöglichen.

12. Werkzeug nach einem der vorhergehenden Ansprüche, wobei der Zylinder (30) einen Durchmesser von 2 cm oder weniger aufweist.

13. Werkzeug nach Anspruch 12, wobei der Zylinder (30) einen Durchmesser von 1,5 cm oder weniger aufweist.

14. Werkzeug nach Anspruch 13, wobei der Zylinder (30) einen Durchmesser von 1,0 cm oder weniger aufweist.

15. Werkzeug nach Anspruch 14, wobei der Zylinder (30) einen Durchmesser von 0,5 cm oder weniger aufweist.

## Revendications

1. Instrument pour l'application d'un produit hémostatique comprenant un corps cylindrique (30) pour contenir une quantité d'un produit hémostatique, une sortie (42) et un piston plongeur (31) pouvant être actionné pour expulser le produit hémostatique du corps cylindrique, le corps cylindrique a un manchon interne (36) et un manchon externe (38), **caractérisé en ce qu'**une ouverture de chargement (37, 39) est prévue dans chacun des manchons, les manchons pouvant tourner l'un par rapport à l'autre, moyennant quoi les ouvertures peuvent être placées, en alternance, en alignement ou hors d'alignement l'un par rapport à l'autre ; un piston plongeur (31) qui peut être déplacé axialement par rapport au corps cylindrique entre une position retirée et une position complètement insérée, et une sortie (42) au niveau d'une extrémité (41) du corps cylindrique (30) opposée au piston plongeur (31), ladite sortie ayant un diamètre au moins minimalement plus petit que le diamètre interne du corps cylindrique (30), pour fournir une butée afin de limiter l'insertion complète du piston plongeur (31).

2. Instrument selon la revendication 1, dans lequel les ouvertures de chargement, lorsqu'elles sont alignées, contiennent une chambre à l'intérieur du piston plongeur (30), pour y placer un produit hémostatique, lorsque le piston plongeur (31) est complètement retiré.

3. Instrument selon la revendication 1 ou 2, dans lequel la sortie (42) est définie par un rebord interne (43) autour de l'extrémité respective du corps cylindrique (30) qui fournit un échelon pour empêcher le piston plongeur (31) d'être poussé au-delà de l'extrémité respective du corps cylindrique (30).

4. Instrument selon l'une quelconque des revendications 1 à 3, dans lequel les ouvertures de chargement comprennent des fentes allongées (37, 39), une dans chacun des manchons interne et externe (36, 38), le manchon interne (36) étant fixé par rapport à un élément d'extrémité qui a une ouverture pour faire coulisser le piston plongeur (31).

5. Instrument selon l'une quelconque des revendications 1 à 4, dans lequel la sortie (42) est formée comme une buse arrondie, progressivement rétrécie ou conique avec une ouverture de diamètre réduit par rapport au diamètre du corps cylindrique (30).

6. Instrument selon la revendication 5, dans lequel la buse est formée avec des fentes longitudinales dans une matrice cruciforme convergeant sur l'ouverture.

7. Instrument selon l'une quelconque des revendications précédentes, dans lequel la surface externe du corps cylindrique (30) a un revêtement en matériau autolubrifiant ou à faible frottement.

8. Instrument selon l'une quelconque des revendications précédentes, dans lequel le corps cylindrique (30) est calibré de sorte que l'utilisateur peut évaluer la quantité de matière appliquée.

9. Instrument selon l'une quelconque des revendications précédentes, dans lequel le corps cylindrique (30) est réalisé avec un matériau qui est soluble dans les fluides corporels tel que le plasma sanguin.

10. Instrument selon la revendication 1, dans lequel un corps cylindrique (30) à extrémité ouverte est prévu avec un piston plongeur retirable, qui peut être inséré dans l'extrémité ouverte, après une charge de matière hémostatique, le corps cylindrique (30) ayant une extrémité de décharge opposée à ladite extrémité ouverte.

11. Instrument selon la revendication 10, dans lequel l'extrémité de décharge comprend un cône avec des fentes longitudinales qui fournissent des rabats qui peuvent être dilatés pour permettre la sortie d'un corps de matière hémostatique.

12. Instrument selon l'une quelconque des revendications précédentes, dans lequel le corps cylindrique (30) a un diamètre de 2 cm ou moins.

13. Instrument selon la revendication 12, dans lequel le corps cylindrique (30) a un diamètre de 1,5 cm ou moins.

14. Instrument selon la revendication 13, dans lequel le corps cylindrique (30) a un diamètre de 1,0 cm ou moins.

15. Instrument selon la revendication 14, dans lequel le corps cylindrique (30) a un diamètre de 0,5 cm ou moins.
